# EUROPEAN PATENT SPECIFICATION

(11) **EP 1 565 479 B1**
(45) Date of publication and mention of the grant of the patent: **14.06.2006**
(21) Application number: 03780024.0
(22) Date of filing: 21.11.2003
(51) Int. Cl.: C07H 19/00, C07H 21/00, C07F 7/18

(54) **2'-O-TRISUBSTITUTED SILYLOXYMETHYL-RIBONUCLEOSIDE-DERIVATIVE AND METHOD FOR PREPARING THE SAME**
2'-O-TRISUBSTITUIERTER-SILYLOXYMETHYL-RIBONUKLEOSID-DERIVAT UND VERFAHREN ZU SEINER HERSTELLUNG
DERIVE DU RIBONUCLEOSIDE 2'O-SILYLOXYMETHYL TRISUBSTITUE ET SON PROCEDE DE PREPARATION

(30) Priority: 22.11.2002 GB 0227352
(43) Date of publication of application: 24.08.2005
(73) Proprietor: Novartis AG, 4056 Basel (CH); Novartis Pharma GmbH, 1230 Wien (AT)
(72) Inventor: NATT, François, Jean-Charles, CH-4147 Aesch (CH); HUNZIKER, Jürg, CH-5000 Aarau (CH); HALL, Jonathan, CH-4143 Dornach (CH); MARTIN, Pierre, CH-4310 Rheinfelden (CH)
(74) Representative: Morris, Clive Sydney
(86) International application number: PCT/EP2003/013113
(87) International publication number: WO 2004/049274

(56) References cited:
- WO-A-00/08042
- WO-A-99/09044
- GUNDERSEN ET AL: "Chloromethoxysilanes as protecting reagents for sterically hinered alcohols" ACTA CHEMICA SCANDINAVICA, MUNKSGAARD, COPENHAGEN, DK, vol. 43, no. 7, 1989, pages 706-709, XP002086775 ISSN: 0904-213X cited in the application

## Description

### FIELD OF THE INVENTION

The invention is in the field of nucleic acid chemistry and concerns methods for the preparation and use of ribonucleoside derivatives with novel protecting groups. The inventive compounds are particularly adapted for the automated preparation of oligoribonucleotides.

### BACKGROUND OF THE INVENTION

Applications for synthetic nucleic acids are numerous and key for the understanding of biological processes. Among these applications, the use of synthetic oligonucleotides for the specific down-regulation of proteins by specific hybridisation in the cell of the synthetic oligonucleotide to a mRNA is known as an antisense mechanism and has been widely described (1). More recently, RNA interference, a technique using dsRNA known as siRNA, has been successfully used to inhibit the translation of mammalian mRNA to its protein (2). The great promise of RNA technology has created a need for the development of efficient and cost effective preparation of synthetic oligoribonucleotides.
Oligoribonucleotide synthesis is more challenging than oligodeoxynucleotide synthesis, mainly because of the 2'-OH group which is present in ribonucleic acids, but not in deoxyribonucleic acids. Chemical synthesis of oligoribonucleotides is normally based on a protected ribonucleoside derivative immobilized on a solid phase to which further protected ribonucleotide derivatives are coupled in consecutive steps of one synthesis cycle each until the desired length of chain is achieved. To ensure an efficient synthesis and to avoid RNA degradation during the preparation process, the protecting group strategy for the 2'-OH group should be perfectly orthogonal with that of other protecting groups and the protecting group should be removed as late as possible in the process. So far, mainly the following types of protection groups have been used to protect the 2'-OH group:
2'-O-TBDMS chemistry is the commonly used protecting group for RNA synthesis (3). It is orthogonal with other protecting groups. However, 2'-3' phosphoryl migration during oligoribonucleotide synthesis has been reported (4). Moreover, steric hindrance of t-butyldimethylsilyl group close to reactive phosphoramidite significantly diminishes coupling efficiency. The latter limitation can be reduced but at the price of longer coupling times, the use of higher molar excesses of reagents and special phosphoramidite activators like 5-(Benzylmercapto)-1 H-tetrazole for instance (5).

2'-O-ACE chemistry has been described by Caruthers et al. (6) as an alternative to TBDMS chemistry. There, 2'-OH is protected by an acid labile orthoester. As compared to TBDMS,' lower hindrance of that protecting group allows higher coupling rates. The acid lability of 2' orthoester requires a non-acid labile temporary protection of the 5'-OH. This was accomplished with trisubstituted silyl groups which are removed at the end of each coupling cycle by a fluoride-containing solution. Consequently, reagents for the preparation of 2'-O-ACE building blocks have to be scaled up specifically. Such 5' deprotection may in some cases be problematic: it requires a dedicated synthesizer resistant to fluoride ions and the use of the commonly used silica based supports (like Controlled Pore Glass) is not possible. 2'-O-TOM chemistry has been reported by Pitsch et al. (6). As for 2'-OTBDMS protecting group strategy, 2'-O-TOM protecting group is removed upon treatment with fluoride ions. Originally, it was developed to allow the synthesis of long RNA without the 2'-3' phosphoryl migration observed with 2'-O-TBDMS. In this case, due to the acetal nature of the bond between nucleoside and protection-group, no migration of the protection-group to a different position in the ribonucleoside-derivative, in particular to the neighbouring 3'-O-position, can occur. Such isomerization is a well known problem in the synthesis of the conventional 2'-O-silyl-substituted RNA-units.
An additional important advantage of this protecting group is the lower hindrance of the protecting group due to the acetal spacer between 2'-oxygen and the bulky triisopropylsilyl group.

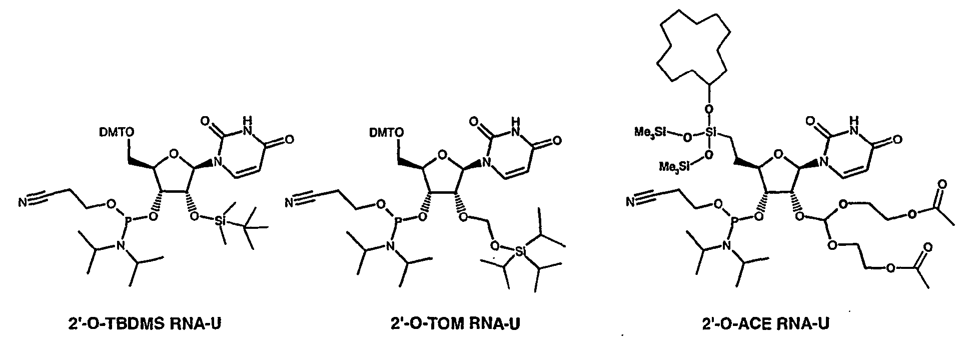

Both 2'-O-TOM and 2'-O-ACE are affording coupling yields approaching those observed in oligodeoxynucleotide synthesis. Satisfying coupling yields are also obtainable with 2'-O-TBDMS chemistry but at price of use of unusual activators or of higher molar excesses of building blocks. In all cases, the building blocks mentioned are contributing to a large extent to the manufacturing costs of oligoribonucleotides. Secondly, post-synthetic processing of oligoribonucleotides is more demanding as compared to processing of oligodeoxyribonucleotides. The latter aspect can be of special importance when high-throughput demand has to be satisfied.
There is a need for improved RNA building blocks which are easily affordable and allowing an easier post-synthetic processing.
Substituted silyloxymethyl groups have been used as protecting groups of hydroxyl groups in the past (7,8). Steric hindrance of substituents on Si atom modulates stability and removal conditions of the protecting group. For instance, silyloxymethyl bearing tertiary carbons vicinal to the Si atom have been reported (7), in these cases, yields for removal of protecting groups were suboptimal as compared to those usually observed with TBDMS protection, and apparently not suited for solid phase oligoribonucleotide synthesis.

The present invention now provides novel and improved groups for the protection at the 2'-OH position of ribonucleosides derivatives that are particularly suited for automated oligoribonucleotide solid phase synthesis. A new procedure for the preparation of these building blocks is provided. Finally, the use of these building blocks in RNA solid phase synthesis is disclosed.

### SUMMARY OF THE INVENTION

In one aspect, the present invention provides ribonucleoside-derivatives of the formula 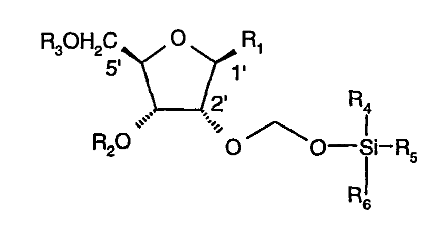 wherein
R₁ is a base of the purine- or pyrimidine-family or a derivative of such a base or any other residue which serves as a nucleobase surrogate,
R₂ is a proton or a substituted derivative of phosphonic acid,
R₃ is a proton or a protection-group for the oxygen atom in 5'-position,
R₄, R₅ and R₆ are independently alkyl or aryl or a combination of alkyl and aryl or heteroatom, R₄, R₅ or R₆ may also be cyclically connected to each other;
and
wherein at least one of the R₄, R₅ or R₆ substituents comprises a tertiary C-atom or a heteroatom vicinal to the Si-atom.

In a preferred aspect, the substituent comprising the tertiary C-atom vicinal to the Si-atom comprises from 4 to 24 C-atoms, more preferably from 5 to 24 C-atoms and yet more preferably from 6 to 24 C-atoms. In a more preferred aspect, the substituent comprising the tertiary C-atom vicinal to the Si-atom is an alkyl-substituent selected from the group consisting of tert-butyl, tert-pentyl, tert-hexyl, tert-heptyl, tert-octyl, tert-nonyl, tert-decyl, tert-undecyl, tert-dodecyl. In a another preferred aspect, the substituent comprising the tertiary C-atom vicinal to the Si-atom is selected from the group of 1,1-dimethyl ethyl, 1,1-dimethyl-propyl, 1,1-dimethyl-butyl, 1,1-dimethyl-pentyl, 1,1-dimethyl-hexyl, thexyl (1,1,2-trimethyl-propyl), 1,1,2-trimethyl-butyl, 1,1,2-trimethyl-pentyl, 1,1,2-trimethyl-hexyl, 1,1,2,2 tetramethyl-propyl, 1,1,2,2-tetramethyl-butyl. In a more preferred embodiment, the substituents of the above groups comprises at least 5 C-atoms, more preferably at least 6 C-atoms.

In a related aspect, the present invention provides ribonucleoside-derivatives wherein the substituent vicinal to the Si-atom comprises a substituted heteroatom. In a preferred aspect, the substituent vicinal to the Si-atom comprises a substituted bivalent heteroatom, in a more preferred aspect this substituent is oxygen.

In another aspect, the present invention provides a method for the preparation of a ribonucleoside-derivatives, comprising reacting a nucleoside with the formula 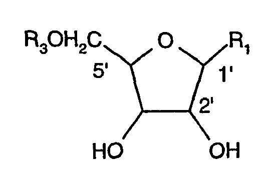 where R₁ and R₃ are as defined as above, with a silyloxymethyl derivative of the formula 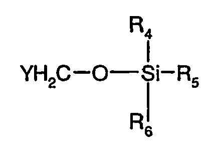 wherein Y is a suitable leaving group and wherein R₄, R₅ and R₆ are independently alkyl or aryl or a combination of alkyl and aryl or a heteroatom, R₄, R₅ or R₆ may also be cyclically connected to each other. In a preferred embodiment Y is halogen. In another preferred embodiment, R₄, R₅ and R₆ together comprise between 6 and 30 carbon atoms. In a further preferred embodiment, R₄, R₅ and R₆ comprise at least one substituted heteroatom vicinal to Si atom, which is preferably a bivalent atom, more preferably oxygen. The ribonucleoside derivative may further be substituted on the oxygen in 3'-position with a group comprising of a derivative of phosphonic acid.

Another aspect of the present invention provides a method for the preparation of a ribonucleoside-derivative, comprising reacting a ribonucleoside derivative with the formula 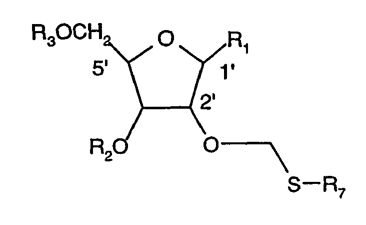 upon an electrophilic activation with a compound of formula: 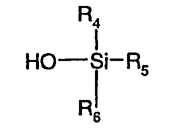 wherein R₁ is defined as above and R₇ is a alkyl- or aryl-group, or alkyl-aryl-group,
wherein R₂ is a protecting group,
wherein R₃ is a protecting group,
wherein R₄, R₅ and R₆ are defined as above.

In a preferred embodiment, the ribonucleoside derivative is further substituted on the oxygen in 3'-position with a group comprising of a derivative of phosphonic acid.

### DETAILED DESCRIPTION OF THE INVENTION

### Abbreviations:

- TBDMS: t-butyldimethylsilyl
- ACE: bis[2-(acetyloxy)ethoxy]methyl
- TOM: (triisopropylsilyl)oxymethyl
- THEX: [((1,1,2-trimethyl-propyl)-dimethylsilyl)]-oxymethyl
- DCA: Dichloroacetic acid
- dsRNA: Double-stranded RNA
- siRNA: Small interfering RNA

The present invention relates to 2'-O-silyloxymethyl ribonucleotide-derivatives for application in the chemical synthesis of ribonucleic acids comprising a D or L-ribose unit having the following general structural formula: 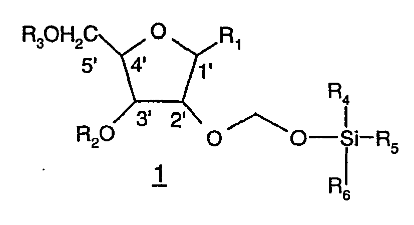 whereby
R₁ is a base of the purine- or pyrimidine-family or a derivative of such a base or any other residue which serves as a nucleobase surrogate, R₂ is a proton or a substituted derivative of phosphonic acid, R₃ is a proton or a protection-group for the oxygen atom in 5'-position, and R₄, R₅ and R₆ are independently alkyl- or aryl-groups or a alkyl-aryl-group. R₄, R₅ or R₆ may also be cyclically connected to each other.

The protection group R₃ in 5'-O-position is e.g. a monomethoxytrityl- or dimethoxytrityl-group or a different, suitable group which is removed from the growing sequence during chain building such freeing a bonding position for coupling the next unit to be added to the chain.

The base component R₁ of the ribonucleoside derivative is preferably a base of the purine or pyrimidine family, e.g. one of the five nucleobase adenine, cytosine, thymine, uracil, guanine or a derivative thereof, or any other residue which serves as a nucleobase surrogate. It can be protected by an acyl-substituent which can be removed after chain creation.

In the 3'-O-position, R₂ is a derivative of phosphonic acid, such as an N,N- and O-substituted phosphoramidite group, whereby the N-substituents are alkyl- or aryl-groups which can be further substituted and /or cyclically connected to each other. By activation of the nitrogen of the disubstitued amino-group the phosphorus centre is activated for coupling the unit to a growing chain.

This invention now provides new and advantageous 2'-O-silyloxymethyl protecting groups wherein R₄, R₅ or R₆ is independently an alkyl- or aryl-substituent, or an alkyl-aryl- or aryl-alkyl or a substituted heteroatom substituent, and
wherein at least one of the R₄, R₅ or R₆ substituents comprises a heteroatom or a tertiary C-atom as can be represented by the formula 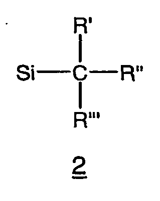 wherein R', R" and R"' are alkyl- or aryl, or an alkyl-aryl-substituent- or aryl-alkyl or a substituted heteroatom, and wherein R', R" and R"' are not H. R', R" and R"' may be the same or different, preferred are substituents comprising 1 to 12 C-atoms, preferably 1 to 6 C-atoms and more preferably are 1 to 4 C-atoms. R', R" and R"' may also be cyclically connected to each other, for instance R' may be cyclically connected to R" or R"', or R" may be cyclically connected to R"'. In a preferred embodiment, two of the substituents are identical and comprise from 1 to 6 C-atoms, preferably from 1 to 4 C-atoms. The third substituent comprises preferably at least 3 C-atoms, preferred are from 3 to 12 C-atoms, more preferred are from 3 to 6 C-atoms.

Thus, in one embodiment at least one of the substituents R₄, R₅ and/or R₆ is (C₄ to C₂₄)-tertiary-alkyl and/or aryl, preferably (C₅ to C₁₈)-tertiary-alkyl and/or aryl, more preferably (C₆ to C₁₂)-tertiary-alkyl and/or aryl, wherein the tertiary C-atom is vicinal to the Si-atom. Without intending to be limited to these groups, examples of such substituents may comprise for instance tert-butyl, tert-pentyl, tert-hexyl, tert-heptyl, tert-octyl, tert-nonyl, tert-decyl, tert-undecyl, tert-dodecyl, thexyl (1,1,2-trimethyl-propyl), 1,1,2-trimethyl-butyl, 1,1,2-trimethyl-pentyl, 1,1,2-trimethyl-hexyl, 1,1,2,2 tetramethyl-propyl, 1,1,2,2-tetramethyl-butyl. In a preferred embodiment, the substituent is tert-pentyl or higher, in a more preferred embodiment the substituent is tert-hexyl or higher. More preferred examples comprise e.g. 1,1 dimethyl-ethyl, 1,1-dimethyl-propyl, 1,1-dimethyl-butyl, 1,1-dimethyl-pentyl, 1,1-dimethyl-hexyl, 1,1,2-trimethyl-propyl, 1,1,2-trimethyl-butyl, 1,1,2-trimethyl-pentyl, 1,1,2-trimethyl-hexyl, 1,1,2,2 tetramethyl-propyl, 1,1,2,2-tetramethyl-butyl.ln another embodiment R₄, R₅ and/or R₆ comprise a heteroatom.

The substituent(s) which do not comprise a tertiary C-atom may be identical or different substituents. These substituents are preferably alkyl- or aryl- substituents, or alkyl-aryl-substituents. Preferred are substituent comprising from 1 to 12 C-atoms, preferably from 1 to 8 C-atoms, more preferably from 1 to 4 C-atoms. Without intending to be limited to these groups, examples of such substituents may comprise for instance methyl, ethyl, propyl, butyl, pentyl, hexyl, i-propyl, sec-butyl, isobutyl, sec-pentyl.

In another embodiment, R₄, R₅ and/or R₆ comprise a substituted heteroatom like for instance silicon, germanium, tin, lead, nitrogen, oxygen, sulfur such as for instance can be represented for a "four-valent" heteroatom by the formula: 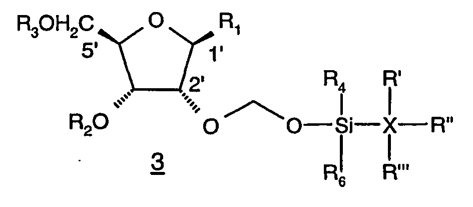 wherein X is Si or Ge, Sn or Pb and wherein R', R" and R"' are defined as for formula 2, and R₄ and R₆ are defined as above. Any of the substituents R₄, R₅ or R₆ may comprise the heteroatom vicinal to the Si-atom, preferred only one as is exemplified in formula 3. For "bivalent" heteroatoms like oxygen, or for trivalent heteroatoms like nitrogen formula 3 may be adapted accordingly as a person of skill in the art would readily recognize. In a preferred embodiment of the present invention, X is oxygen.

The compound may be prepared by methods known in the art such as via the organometallic route. This route is described for instance in WO99/09044 (6). The reaction 4 → 5a/5b → 6 shows an example of the preparation of a compound of the present invention via the organometallic route. Briefly, a ribonucleoside protected at the 5'-O is reacted with e.g. chloromethyl[dimethyl-(1,1,2-trimethylpropyl)silyl]ether (or THEX-Cl) in the presence of a suitable organometallic salt, such as for instance dibutyltindichloride or dibutyltinoxide. THEX-Cl itself is prepared in a manner similar to TOM-Cl according to a published procedure (7). However, unlike TOM-Cl, the preparation of THEX-Cl does not require a final distillation step of the reagent prior to the reaction with the ribonucleoside which represents a significant simplification. In the reaction of THEX-Cl with a 5'-O-protected ribonucleoside a mixture of 2'- and 3'-protected ribonucleosides is obtained, wherefrom the 2'-substituted ribonucleoside is purified by for instance chromatographic means. In a subsequent step the 3'-OH group of the purified compound 5a is converted to the phosphoramidite 6 according to methods known in the art (Sinha, N.D. et al., Tetrahedron Lett. 1983, 24, 5843; Sinha, N.D. et al., Nucleic Acid Res. 1984, 12, 4539).
This method requires the use of substituted silyloxymethyl ethers wherein substituents contain alkyl, aryl, or arylalkyl groups and which can be cyclically connected. In a further embodiment, this method is applicable to silyloxymethylethers wherein at least one of the substituents contains at least one substituted heteroatom like for instance silicon, germanium, tin, lead, nitrogen, oxygen, sulfur

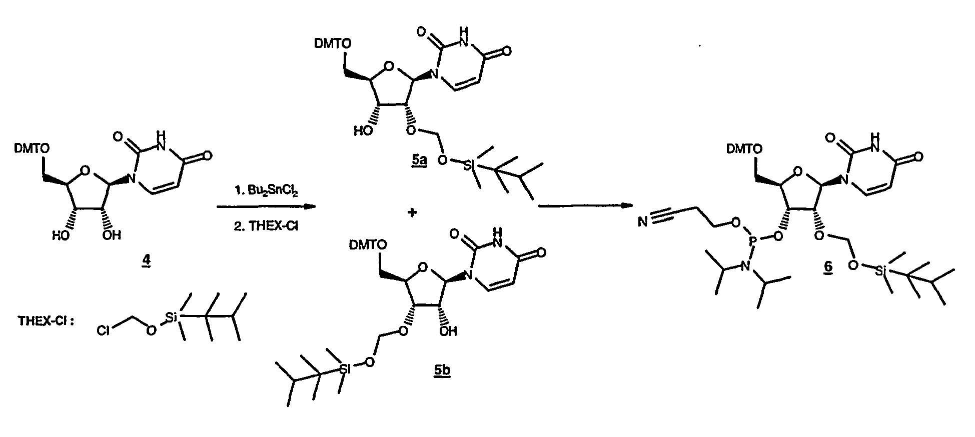

The reaction may be carried out in solution or on solid phase or by using polymer supported reagents. The solvent can be a hydrocarbon solvent, ethereal solvent, nitrile solvent, chlorinated solvent, heterocyclic solvent, sulfoxide solvents, etc.. Specific examples of suitable solvents include pyridine, N,N-dimethylformamide (DMF), tetrahydrofuran (THF), dimethylsulfoxide (DMSO), acetonitrile, dichloroethane and methylene chloride. Preferrably, dichloroethane is used.

Although the reaction may be carried out at room temperature, it may also be carried out at a temperature range of 0 to 150°C preferably at 10 to 100°C.

In a further aspect of the present invention, the compounds of the invention are prepared by a new and superior method as exemplified by the reaction 7 → 8 → 9 → 10 → 6.

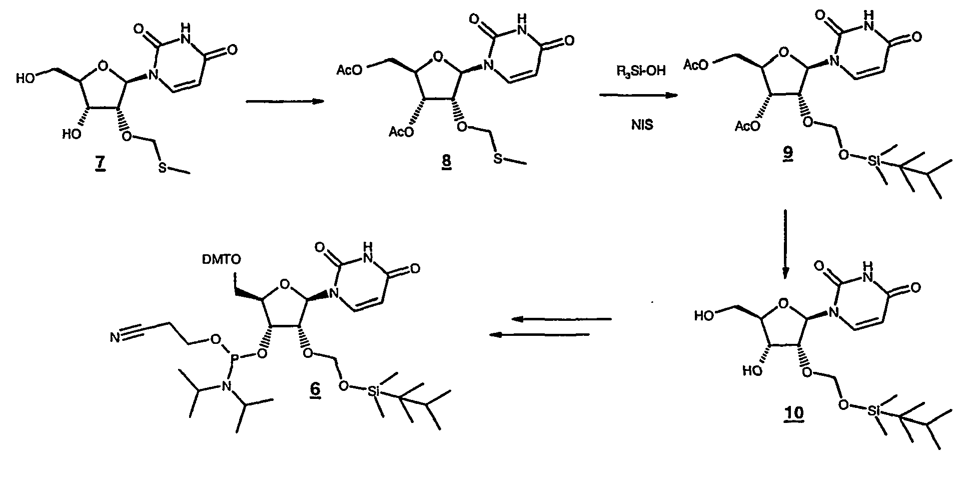

This method allows the selective introduction of 2'-OH protecting groups via first introducing a 2'-O-alkylthiomethyl, arylthiomethyl, alkylarylthiomethyl or arylalkylthiomethyl group. It avoids the unselective step described in the organometallic route. This new method is generally applicable for the introduction of oxymethyl derivatives selectively on 2'-OH group of ribonucleosides and is not restricted to the introduction of protecting groups as described above. The methods for the preparation of protected ribonucleotides currently known in the art, such as the organometallic route, are not 2'-3' selective for the introduction of the protecting group. This method allows the selective introduction of the methylthiomethyl group at the 2' position and thereby prevents an unselective step late in the synthesis scheme.

In this new route a 2'-O-alkythiomethyl-ribonucleoside as can be represented by the formula 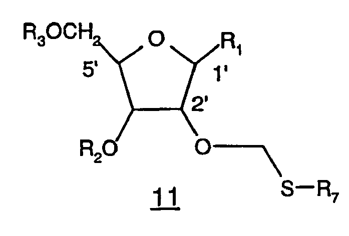 wherein R₇ is alkyl or aryl or a combination of alkyl and aryl, is reacted with a silanol of the general formula HOSiR₄R₅R₆. In a preferred embodiment R₇ is a (C₁ to C₂₀) -, more preferred is (C₁ to C₁₀)- alkyl and/or aryl. In another preferred embodiment R₇ is for instance methyl, ethyl, propyl, butyl, pentyl, hexyl, iso-propyl, sec-butyl, iso-butyl, sec-pentyl. The substituents R₄, R₅ and R₆ of the silanol are identical or different alkyl or aryl or a combination of alkyl and aryl substituents, or substituted heteroatoms and which may also cyclically be connected to each other. In a preferred embodiment the three substituents together comprise between 3 and 30 carbon atoms each.

In another preferred embodiment, compound 11 is reacted with a silanol of the general formula HOSiR₄R₅R₆ with the substituents R₄, R₅, R₆ as defined above, under suitable conditions known in the art. These comprise an electrophilic activating reagent or reagent combination such as, but not limited to, for instance N-halosuccinimides and a catalytic amount of an acid. The reaction may be carried out in solution or on solid phase or by using polymer supported reagents. The solvent can be a hydrocarbon solvent, ethereal solvent, nitrile solvent, chlorinated solvent, heterocyclic solvent, sulfoxide solvents, etc.. Specific examples of suitable solvents include pyridine, N,N-dimethylformamide (DMF), tetrahydrofuran (THF), dimethylsulfoxide (DMSO), acetonitrile, dichloroethane and methylene chloride. Preferrably, dichloromethane is used. Although the reaction may be carried out at room temperature, it may also be carried out at a temperature of -78°C to 100°C preferably at 0 to 50°C.
The alkylthiomethyl-substituent at the 2'-OH group comprises preferably a (C₁ to C₁₂)-alkyl and/or aryl group, preferably a (C₁ to C₆)-alkyl and/or aryl group. Examples of preferred substituents comprise methylthiomethyl, ethylthiomethyl, propylthiomethyl, isopropylthiomethyl or butylthiomethyl.

The following Examples illustrate the present invention, without in any way limiting the scope thereof.

### EXAMPLES

### 1. Preparation of THEX building blocks via organometallic route

Scheme 1 depicts the synthetic scheme for the introduction of the THEX protecting group on 5'-O-DMTr Uridine and the subsequent phosphitylation.

### Preparation of (1,1,2-trimethyl-propyl)-dimethylsilyloxymethyl chloride (THEX-CI)

A suspension of 11.1ml (0.15mol) ethanethiol and 4.5g (0.15mol) para-formaldehyde was treated with two drops of NaOMe/MeOH (30%) and stirred 1 h at 40°C. After cooling, 150ml CH₂Cl₂ and 22.66g (0.333mol) imidazole were added. After 10 minutes, 32.66g (0.167mol) (1,1,2-trimethyl-propyl)-dimethylsilyl chloride was added dropwise. The resulting suspension was stirred at room temperature for 24 hours and diluted with 300ml n- hexane. After adding 200ml 2M NaH₂PO₄ solution,stirring(15 minutes) and phase separation, the organic phase was dried over Na₂SO₄ and evaporated. The residue was dissolved in 100m1 CH₂Cl₂, treated dropwise with 12.3ml (20.4g, 0.152mol) sulfurylchloride in 50ml CH₂Cl₂. After 1 hour, the mixture was evaporated. The product was obtained (31.5g) as wax.
¹H-NMR (400 MHz, CDCl₃): 0.5 (s,6H,SiMe₂); 0.65 (12H,CH₃); 1.40 (sept,1H,CH); 5.43 (s,2H,CH₂).

### Preparation of 1-[5'-O-(4,4'-Dimethoxytrityl)-2'-O-[((1,1,2-trimethyl-propyl)-dimethylsilyl)]-oxymethyl-beta-D-ribofuranosyll-uracil (5a)

A solution of 9.5g (17.4mmol) 5'-O-dimethoxytritylated uridine(1) in 200ml 1,2-dichloroethane was treated with 11.23g (87mmol) Huenig's base and then with 5.81 g (19.2mmol) dibutyltindichloride. After 30 minutes, the mixture was heated to 80°C, treated with 4.2g (22.6mmol) (1,1,2-trimethyl-propyl)-dimethylsilyloxymethyl chloride (THEX-Cl) in 50ml dichloroethane and stirred two hours at 80°C. After cooling, the mixture was diluted with 400ml CH₂Cl₂ and 350ml aqueous saturated NaHCO₃ solution were added. After stirring 30 minutes, the layers were separated and the organic layer was evaporated. The residue was chromatographed on silica gel, using ethylacetate/hexane(3:1) containing 0.1% N-methylmorpholine. The product was obtained as solid foam (4.52g).
¹H-NMR (400 MHz, CDCl₃): 0.1 (s,6H,CH₃); 0.6-0.8 (s and d,12H,CH₃); 1.45 (m,1H,CH); 3.15 (d,2H,CH₂); 3.64 (s,6H,OCH₃); 3.85 (q,2H,CH₂); 4.05 (m,1H,CH); 4.15 (m,1H,CH); 4.80 (q,2H,CH₂); 5.12 (d,1H,OH); 5.30 (q,1H,CH); 5.78 d,1H CH); 6.8-5.3 (m,13H); 7.6 (d.1H).

### Preparation of 1-[5'-O-(4,4'-Dimethoxytrityl)-2'-O-[((1,1,2-trimethyl-propyl)-dimethylsilyl)]-oxymethyl-beta-D-ribofuranosyl]-uracil-3'-(2-cyanoethyldiisopropyl)phosphoramidite(6)

A solution of 4.0g (5.56mmol) protected uridine(2), 1.14g (6.68mmol) diisopropylaminotetrazolid and 2.01 g (6.68mmol) bis(N,N-diisopropylamino)-2-cyanoethoxy phosphine in 150ml CH₂Cl₂ were stirred 24 hours at roomtemperatur. The mixture was diluted with 100ml CH₂Cl₂ and washed twice with 50ml aqueous saturated NaHCO₃ solution. The dried (Na₂SO₄) organic phase was evaporated and the residue was subjected to column chromatography (ethylacetate/hexane 3:2 with additional 0.1% N-methylmorpholine). The product was obtained as a solid foam (4.12g).
³¹P-NMR (400 MHz, CDCl₃): 151.183 (s) and 151.537 (s).

### 2. Preparation of THEX building blocks via 2'-O-methylthiomethyl route 3',5'-O-Diacetyl-2'-O-methylthiomethyl-uridine(8)

A solution of 4.53g (14.8mmol) 2'-O-methylthiomethyl uridine 7 (8) in 50ml pyridin was treated with 3.04g (29.7mmol) Ac₂O. After 24h stirring, the solution was evaporated. The residue was dissolved in 40ml EtOAc, washed with water and dried with Na₂SO₄. After evaporation, the pure title-compound was obtained (5.05g).

### 3',5'-O-Diacetyl-2-O-[((1,1,2-trimethyl-propyl)-dimethylsilyl)-oxymethyl]-uridine (9)

0.33g (1.47mmol) N-iodosuccinimid in 3 ml THF was added to a solution of 0.5g (1.287mmol) 8, 0.978g (6.lmmol) (1,1,2-trimethyl-propyl)-dimethyl silanol, 10ml CH₂Cl₂ and 1 drop MeOSO₃H. After 2h stirring, 2ml NaHSO₃ (37%) was added, then 100ml CH₂Cl₂. The organic layer was separated and dried with Na₂SO₄. After filtration, the solution was evaporated: 552mg pure title-compound.

### 2'-O-[((1,1,2-trimethyl-propyl)-dimethylsilyl)-oxymethyl]-uridine(10)

A solution of 187mg (0.37mmol) 9, 20ml MeOH and 0.135 ml (0.74mmol) NaOMe/MeOH (30%) was stirred 30min. at OoC. After evaporation, the residue was filtered through a small silica gel column (EtOAc/MeOH 4:1): 140mg 10 as powder.

### 3. Procedure for the incorporation of 6 into oligodeoxynucleotide by phosphoramidite chemistry and fast deprotection thereof

Oligonucleotide synthesis were typically performed on an ABI394 automated DNA synthesizer (Applied Biosystems). DNA Phosphoramidites, THEX protected Uridine phosphoramidite (6) or TOM protected Uridine phosphoramidite (Xeragon, Inc.) were dissolved in dry acetonitrile at a 5% w/v concentration; coupling was made by activation of phosphoramidites using a 0.2 M solution of benzimidazolium triflate (9) in acetonitrile. Coupling times were between 1-5 minutes. A first capping was made using standard capping reagents. Oxidation was made using an 0.1M iodine solution in THF/water/pyridine (1:1:1). A second capping was performed after oxidation. Detritylation before the next coupling was effected with 2% dichloroacetic acid in dichloroethane.

Upon completion of oligonucleotide chain elongation, the solid support was transferred to an Eppendorf tube.

When prepared with THEX protected Uridine phosphoramidite (6), oligonucleotides were cleaved from support and deprotected as follows:
1. 32% aq. Ammonia/EtOH 3:1 (250 µl for 0.2 µmole scale), room temperature, 2h lyophilisation to dryness.
2. 1M tetrabutylammonium fluoride in THF (250 µl for 0.2 µmole scale), 30 min at room temperature.
3. 1 M Tris.HCl, pH=7.4 (250 µl for 0.2 µmole scale).

When prepared with TOM protected Uridine phosphoramidite, oligonucleotides were cleaved from support and deprotected as follows:
1. 32% aq. Ammonia/EtOH 3:1 (250 µl for 0.2 µmole scale), room temperature, 2h lyophilisation to dryness.
2. 1 M tetrabutylammonium fluoride in THF (250 µl for 0.2 µmole scale), 6h min at room temperature.
3. 1 M Tris.HCl, pH=7.4 (250 µl for 0.2 µmole scale).

Resulting crude solutions were analysed by Capillary Gel Electrophoresis.

Results are summarized in table 1

| # | Sequence | TOM (purity%) | THEX (purity%) |
|---|---|---|---|
| 11 | TTT TTU TTT TTT TTT | 85 | 79 |
| 12 | TTT TTU UUU TTT TTT | 67 | 72 |

As shown in table 1, and figures 1-4, quality of crude material obtained with THEX protected Uridine phosphoramidite 6 and TOM protected Uridine phosphoramidite are very similar.
Use of 2'-O-THEX protecting group strategy allowed the reduction of 2' deprotection from 6h at 35°C (as reported in ref. 6) to 30 min at room temperature.

### References

1. De Mesmaeker, A., Haener, R., Martin, P., Moser, H.E. Acc. *Chem. Res.* 28 (1995) 366 and Bennett, C. Frank, Cowsert, Lex M. *Curr. Opin. Mol. Ther.* 1, (1999), 359.
2. Elbashir, Sayda M.; Harborth, Jens; Lendeckel, Winfried; Yalcin, Abdullah; Weber, Klaus; Tuschl, Thomas. Duplexes of 21-nucleotide RNAs mediate RNA interference in cultured mammalian cells. Nature (London, United Kingdom) (2001), 411 (6836), 494-498.
3. Usman, N.; Ogilvie, K. K.; Jiang, M. Y.; Cedergren, R. J.. The automated chemical synthesis of long oligoribuncleotides using 2'-O-silylated ribonucleoside 3'-O-phosphoramidites on a controlled-pore glass support: synthesis of a 43-nucleotide sequence similar to the 3'-half molecule of an Escherichia coli formylmethionine tRNA. J. Am. Chem. Soc. (1987), 109(25), 7845-54.
4. Morgan, Michael A.; Kazakov, Sergei A.; Hecht, Sidney M. Phosphoryl migration during the chemical synthesis of RNA. Nucleic Acids Research (1995), 23(19), 3949-53.
5. Welz, Rudiger; Muller, Sabine. 5-(Benzylmercapto)-1H-tetrazole as activator for 2'-O-TBDMS phosphoramidite building blocks in RNA synthesis. Tetrahedron Letters (2002), 43(5), 795-797
6. Pitsch, Stefan; Weiss, Patrick A.; Jenny, Luzi; Stutz, Alfred; Wu, Xiaolin. Reliable chemical synthesis of oligoribonucleotides (RNA) with 2'-O-[(triisopropylsilyl)oxy]methyl(2'-O-tom)-protected phosphoramidites. Helvetica Chimica Acta (2001), 84(12), 3773-3795.
7. Gundersen, Lise Lotte; Benneche, Tore; Undheim, Kjell. Chloromethoxysilanes as protecting reagents for sterically hindered alcohols. Acta Chem. Scand. (1989), 43(7), 706-9.
8. Russian J. Bioorg.Chem.2000,26,327-333.
9. Hayakawa, Yoshihiro; Kataoka, Masanori; Noyori, Ryoji. Benzimidazolium Triflate as an Efficient Promoter for Nucleotide Synthesis via the Phosphoramidite Method. Journal of Organic Chemistry (1996), 61(23), 7996-7997.

## Claims

1. A ribonucleoside-derivative of the formula 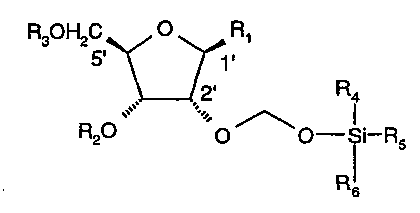 wherein
R₁ is a base of the purine- or pyrimidine-family or a derivative of such a base or any other residue which serves as a nucleobase surrogate,
R₂ is a proton or a substituted derivative of phosphonic acid,
R₃ is a proton or a protection-group for the oxygen atom in 5'-position,
R₄, R₅ and R₆ are independently alkyl or aryl or a combination of alkyl and aryl or heteroatom, R₄, R₅ or R₆ may also be cyclically connected to each other;
and
wherein at least one of the R₄, R₅ or R₆ substituents comprises a tertiary C-atom or a heteroatom vicinal to the Si-atom.

2. A ribonucleoside-derivative according to claim 1 wherein the substituent comprising the tertiary C-atom vicinal to the Si-atom comprises from 4 to 24 C-atoms.

3. A ribonucleoside-derivative according to claim 1 or 2 wherein the substituent comprising the tertiary C-atom vicinal to the Si-atom is an alkyl-substituent selected from the group consisting of tert-butyl, tert-pentyl, tert-hexyl, tert-heptyl, tert-octyl, tert-nonyl, tert-decyl, tert-undecyl, tert-dodecyl.

4. A ribonucleoside-derivative according to claim 1, 2 or 3 wherein the substituent comprising the tertiary C-atom vicinal to the Si-atom is selected from the group of 1,1-dimethyl ethyl, 1,1-dimethyl-propyl, 1,1-dimethyl-butyl, 1,1-dimethyl-pentyl, 1,1-dimethyl-hexyl, 1,1,2-trimethyl-propyl, 1,1,2-trimethyl-butyl, 1,1,2-trimethyl-pentyl, 1,1,2-trimethyl-hexyl, 1,1,2,2 tetramethyl-propyl, 1,1,2,2-tetramethyl-butyl.

5. A ribonucleoside-derivative according to claim 1 wherein the substituent vicinal to the Si-atom comprises a substituted heteroatom.

6. A ribonucleoside-derivative according to claim 5 wherein the substituent vicinal to the Si-atom comprises a substituted bivalent heteroatom.

7. A ribonucleoside-derivative according to claim 6 wherein the heteroatom is oxygen.

8. A method for the preparation of a ribonucleoside-derivative according to claim 1, comprising reacting a nucleoside with the formula 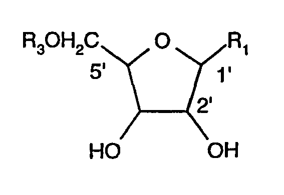 where R₁ and R₃ are as defined in claim 1, with a silyloxymethylderivative of the formula 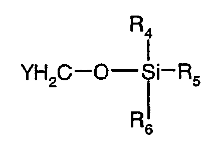 wherein Y is a suitable leaving group
and wherein R₄, R₅ and R₆ are independently alkyl or aryl or a combination of alkyl and aryl or a heteroatom, R₄, R₅ or R₆ may also be cyclically connected to each other.

9. The method of claim 8 wherein Y is a halogen.

10. The method of claim 8 or 9 wherein R₄, R₅ and R₆ together comprise between 3 and 30 carbon atoms.

11. The method of claims 8 or 9 wherein R₄, R₅ or R₆ comprise at least one substituted heteroatom vicinal to Si atom.

12. The method of claim 11 wherein the heteroatom is a bivalent atom.

13. The method of claim 12 wherein the heteroatom is oxygen.

14. The method of claim 11, 12 or 13 wherein the ribonucleoside derivative is further substituted on the oxygen in 3'-position with a group comprising of a derivative of phosphonic acid.

15. A method for the preparation of a ribonucleoside-derivative, comprising reacting a ribonucleoside derivative with the formula 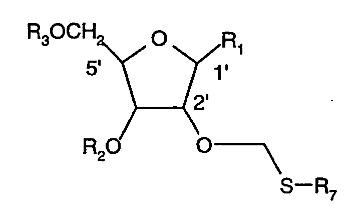 upon an electrophilic activation with a compound of formula: 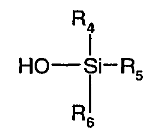 wherein R₁ is defined as in claim 1 and R₇ is a alkyl- or aryl-group, or alkyl-aryl-group,
wherein R₂ is a protecting group,
wherein R₃ is a protecting group,
wherein R₄, R₅ and R₆ are identical or different alkyl or aryl or a combination of alkyl and aryl substituents, which my be further substituted with heteroatoms and which may also cyclically be connected to each other.

16. The method of claim 15 wherein R₄, R₅ and R₆ are defined as in claims 1 to 7.

17. The method of claim 15 or 16 wherein the ribonucleoside derivative is further substituted on the oxygen in 3'-position with a group comprising of a derivative of phosphonic acid.

## Patentansprüche

1. Ribonukleosid-Derivat der folgenden Formel: 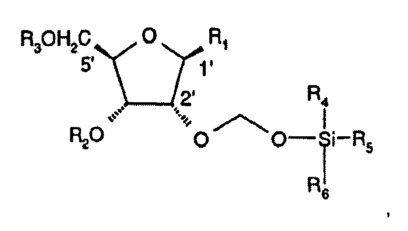 wobei
R₁ für eine Base der Purin- oder Pyrimidin-Familie oder ein Derivat einer solchen Base oder einen beliebigen anderen Rest steht, der als Nukleobasensurrogat dient,
R₂ für ein Proton oder ein substituiertes Derivat der Phosphonsäure steht,
R₃ für ein Proton oder eine Schutzgruppe für das Sauerstoffatom in der 5'-Position steht,
R₄, R₅ und R₆ unabhängig für Alkyl oder Aryl oder eine Kombination von Alkyl und Aryl oder ein Heteroatom stehen, R₄, R₅ und R₆ auch cyclisch miteinander verbunden sein können; und
wobei mindestens einer der R₄-, R₅- oder R₆-Substituenten ein tertiäres C-Atom oder ein Heteroatom benachbart zu dem Si-Atom umfasst.

2. Ribonukleosid-Derivat nach Anspruch 1, wobei der Substituent, der das tertiäre C-Atom benachbart zu dem Si-Atom umfasst, 4 bis 24 C-Atome umfasst.

3. Ribonukleosid-Derivat nach Anspruch 1 oder 2, wobei der Substituent, der das tertiäre C-Atom benachbart zu dem Si-Atom umfasst, für einen Alkylsubstituenten steht, der ausgewählt ist aus der Gruppe, bestehend aus tert.-Butyl, tert.-Pentyl, tert.-Hexyl, tert.-Heptyl, tert.-Octyl, tert.-Nonyl, tert.-Decyl, tert.-Undecyl, tert.-Dodecyl.

4. Ribonukleosid-Derivat nach Anspruch 1, 2 oder 3, wobei der Substituent, der das tertiäre C-Atom benachbart zu dem Si-Atom umfasst, ausgewählt ist aus der Gruppe von 1,1-Dimethylethyl, 1,1-Dimethylpropyl, 1,1-Dimethylbutyl, 1,1-Dimethylpentyl, 1,1-Dimethylhexyl, 1,1,2-Trimethylpropyl, 1,1,2-Trimethylbutyl, 1,1,2-Trimethylpentyl, 1,1,2-Trimethylhexyl, 1,1,2,2-Tetramethylpropyl, 1,1,2,2-Tetramethylbutyl.

5. Ribonukleosid-Derivat nach Anspruch 1, wobei der Substituent benachbart zu dem Si-Atom ein substituiertes Heteroatom umfasst.

6. Ribonukleosid-Derivat nach Anspruch 5, wobei der Substituent benachbart zu dem Si-Atom ein substituiertes zweiwertiges Heteroatom umfasst.

7. Ribonukleosid-Derivat nach Anspruch 6, wobei das Heteroatom für Sauerstoff steht.

8. Verfahren zur Herstellung eines Ribonukleosid-Derivats nach Anspruch 1, wobei das Verfahren umfasst ein Umsetzen eine Nukleosids mit der folgenden Formel 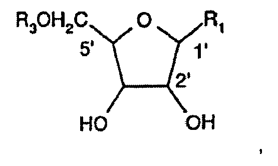 wobei R₁ und R₃ wie in Anspruch 1 definiert sind, mit einem Silyloxymethylderivat der folgenden Formel 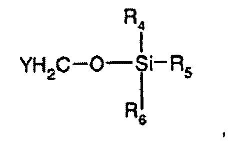 wobei Y für eine geeignete Abgangsgruppe steht und wobei R₄, R₅ und R₆ unabhängig für Alkyl oder Aryl oder eine Kombination von Alkyl und Aryl oder ein Heteroatom stehen, R₄, R₅ oder R₆ auch cyclisch miteinander verbunden sein können.

9. Verfahren nach Anspruch 8, wobei Y für ein Halogen steht.

10. Verfahren nach Anspruch 8 oder 9, wobei R₄, R₅ und R₆ zusammen zwischen 3 und 30 Kohlenstoffatome umfassen.

11. Verfahren nach den Ansprüchen 8 oder 9, wobei R₄, R₅ oder R₆ mindestens ein substituiertes Heteroatom benachbart zu dem Si-Atom umfassen.

12. Verfahren nach Anspruch 11, wobei das Heteroatom für ein zweiwertiges Atom steht.

13. Verfahren nach Anspruch 12, wobei das Heteroatom für Sauerstoff steht.

14. Verfahren nach Anspruch 11, 12 oder 13, wobei das Ribonukleosid-Derivat zusätzlich substituiert ist an dem Sauerstoff in 3'-Position durch eine Gruppe, die ein Derivat der Phosphonsäure umfasst.

15. Verfahren zur Herstellung eines Ribonukleosid-Derivats, wobei das Verfahren umfasst ein Umsetzen eines Ribonukleosid-Derivats mit der folgenden Formel 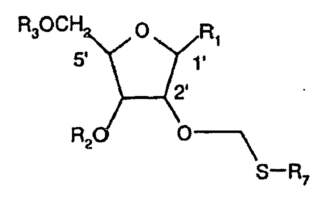 nach einer elektrophilen Aktivierung mit einer Verbindung der Formel: 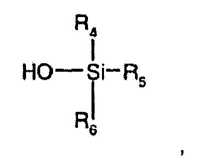 wobei R₁ wie in Anspruch 1 definiert ist und R₇ für eine Alkyl- oder Arylgruppe oder eine Alkylarylgruppe steht,
wobei R₂ für eine Schutzgruppe steht,
wobei R₃ für eine Schutzgruppe steht,
wobei R₄, R₅ und R₆ für identische oder verschiedene Alkyl- oder Aryl- oder eine Kombination von Alkyl- und Aryl-Substituenten stehen, die zusätzlich substituiert sein können durch Heteroatome und die auch cyclisch miteinander verbunden sein können.

16. Verfahren nach Anspruch 15, wobei R₄, R₅ und R₆ wie in den Ansprüchen 1 bis 7 definiert sind.

17. Verfahren nach Anspruch 15 oder 16, wobei das Ribonukleosid-Derivat zuzsätzlich an den Sauerstoff in 3'-Position substituiert ist durch eine Gruppe, die ein Derivat der Phosphonsäure umfasst.

## Revendications

1. Dérivé ribonucléosidique de formule : 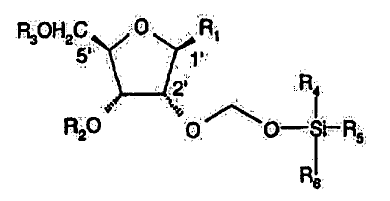 dans laquelle :
R₁ est une base de la famille de la purine ou de la pyrimidine, ou un dérivé d'une telle base, ou tout autre résidu qui sert de substitut d'une nucléobase,
R₂ est un proton ou un dérivé substitué d'acide phosphonique,
R₃ est un proton ou un groupe protecteur pour l'atome d'oxygène en position 5',
R₄, R₅ et R₆ représentent indépendamment un groupe alkyle ou aryle ou une combinaison d'un groupe alkyle et d'un groupe aryle, ou un hétéroatome, R₄, R₅ et R₆ pouvant également être cycliquement connectés les uns aux autres ;
et
dans laquelle au moins l'un des substituants R₄, R₅ et R₆ comprend un atome de C tertiaire ou un hétéroatome proche de l'atome de Si.

2. Dérivé ribonucléosidique selon la revendication 1, dans lequel le substituant comprenant l'atome de C tertiaire proche de l'atome de Si comprend 4 à 24 atomes de C.

3. Dérivé ribonucléosidique selon la revendication 1 ou 2, dans lequel le substituant comprenant l'atome de C tertiaire proche de l'atome de Si est un substituant alkyle choisi dans le groupe constitué par les groupes tert-butyle, tert-pentyle, tert-hexyle, tert-heptyle, tert-octyle, tert-nonyle, tert-décyle, tert-undécyle et tert-dodécyle.

4. Dérivé ribonucléosidique selon la revendication 1, 2 ou 3, dans lequel le substituant comprenant l'atome de C tertiaire proche de l'atome de Si est choisi dans le groupe constitué de : 1,1-diméthyléthyle, 1,1-diméthylpropyle, 1,1-diméthylbutyle, 1,1-diméthylpentyle, 1,1-diméthylhexyle, 1,1,2-triméthylpropyle, 1,1,2-triméthylbutyle, 1,1,2-triméthylpentyle, 1,1,2-triméthylhexyle, 1,1,2,2-tétraméthylpropyle et 1,1,2,2-tétraméthylbutyle.

5. Dérivé ribonucléosidique selon la revendication 1, dans lequel le substituant proche de l'atome de Si comprend un hétéroatome substitué.

6. Dérivé ribonucléosidique selon la revendication 5, dans lequel le substituant proche de l'atome de Si comprend un hétéroatome bivalent substitué.

7. Dérivé ribonucléosidique selon la revendication 6, dans lequel l'hétéroatome est l'oxygène.

8. Procédé de préparation d'un dérivé ribonucléosidique selon la revendication 1, comprenant la réaction d'un nucléoside de formule : 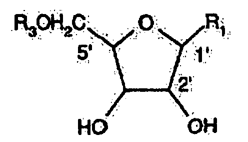 dans laquelle R₁ et R₃ sont tels que définis dans la revendication 1, avec un dérivé de silyloxyméthyle de formule : 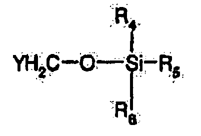 dans laquelle Y est un groupe labile approprié,
et dans laquelle R₄, R₅ et R₆ représentent indépendamment un groupe alkyle ou aryle ou une combinaison d'un groupe alkyle et d'un groupe aryle, ou un hétéroatome, R₄, R₅ et R₆ pouvant également être cycliquement connectés les uns aux autres.

9. Procédé selon la revendication 8, dans lequel Y est un halogène.

10. Procédé selon la revendication 8 ou 9, dans lequel R₄, R₅ et R₆ comprennent ensemble 3 à 30 atomes de carbone.

11. Procédé selon la revendication 8 ou 9, dans lequel R₄, R₅ et R₆ comprennent au moins un hétéroatome substitué proche de l'atome de Si.

12. Procédé selon la revendication 11, dans lequel l'hétéroatome est un atome bivalent.

13. Procédé selon la revendication 12, dans lequel l'hétéroatome est l'oxygène.

14. Procédé selon la revendication 11, 12 ou 13, dans lequel le dérivé ribonucléosidique est davantage substitué sur l'oxygène en position 3' par un groupe comprenant un dérivé d'acide phosphonique.

15. Procédé de préparation d'un dérivé ribonucléosidique, comprenant la réaction d'un dérivé ribonucléosidique de formule : 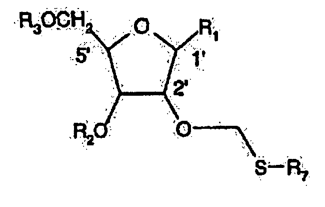 lors d'une activation électrophile, avec un composé de formule : 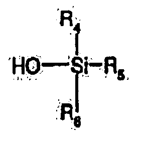 dans laquelle R₁ est tel que défini dans la revendication 1, et R₇ est un groupe alkyle ou aryle ou un groupe alkyl-aryle,
dans laquelle R₂ est un groupe protecteur,
dans laquelle R₃ est un groupe protecteur,
dans laquelle R₄, R₅ et R₆ sont des groupes alkyle ou aryle identiques ou différents, ou une combinaison de substituants alkyle et aryle, qui peuvent être davantage substitués par des hétéroatomes et qui peuvent également être cycliquement connectés les uns aux autres.

16. Procédé selon la revendication 15, dans lequel R₄, R₅ et R₆ sont tels que définis dans les revendications 1 à 7.

17. Procédé selon la revendication 15 ou 16, dans lequel le dérivé ribonucléosidique est davantage substitué sur l'oxygène en position 3' par un groupe comprenant un dérivé d'acide phosphonique.
